# EUROPÄISCHE PATENTSCHRIFT

(11) **EP 3 554 709 B1**
(45) Veröffentlichungstag und Bekanntmachung des Hinweises auf die Patenterteilung: **02.06.2021**
(21) Anmeldenummer: 18708361.3
(22) Anmeldetag: 21.02.2018
(51) Int. Cl.: B01L 3/02

(54) **DISPENSER, SYSTEM UND VERFAHREN ZUM AUFNEHMEN UND ABGEBEN VON FLUIDVOLUMINA**
DISPENSER, SYSTEM AND METHOD TO RECEIVE AND DISPENSE FLUID VOLUMES
DISTRIBUTEUR, SYSTÈME ET MÉTHODE POUR DISTRIBUER OU PRÉLEVER DES VOLUMES DE LIQUIDE

(30) Priorität: 23.02.2017 DE 202017101008 U
(43) Veröffentlichungstag der Anmeldung: 23.10.2019
(73) Patentinhaber: BRAND GMBH + CO KG, 97877 Wertheim (DE)
(72) Erfinder: SETZER, Daniel, 63936 Schneeberg (DE); SCHRAUT, Jürgen, 97855 Lengfurt (DE)
(74) Vertreter: Von Rohr Patentanwälte Partnerschaft mbB
(86) Internationale Anmeldenummer: PCT/EP2018/054289
(87) Internationale Veröffentlichungsnummer: WO 2018/153924

(56) Entgegenhaltungen:
- EP-A2- 0 576 967
- US-A- 4 567 780
- US-A- 4 821 586
- US-A- 5 998 218

## Beschreibung

Die Erfindung betrifft einen Dispenser zum Aufnehmen und Abgeben von Fluidvolumina gemäß dem Oberbegriff von Anspruch 1. Die Erfindung betrifft außerdem ein System zum Aufnehmen und Abgeben von Fluidvolumina gemäß Anspruch 8 und ein Verfahren zum Aufnehmen und Abgeben von Fluidvolumina gemäß Anspruch 9.

Aufgabe von Systemen der in Rede stehenden Art ist es, aus einem Behälter ein Fluidvolumen aufzunehmen und anschließend in einen anderen oder mehrere andere Behälter abzugeben. Solche Systeme dienen insbesondere zum wiederholten Dispensieren, Titrieren bzw. Pipettieren von Flüssigkeiten.

Solche Systeme umfassen einen Dispenser und eine als Austauschteil ausgebildete Kolben-Zylinder-Einheit, die am Dispenser lösbar anbringbar, insbesondere einsetzbar bzw. einlegbar, ist. Nach einem oder mehreren Dispensiervorgängen kann die Kolben-Zylinder-Einheit von dem Dispenser gelöst werden. Anschließend kann eine andere, insbesondere unterschiedliche Kolben-Zylinder-Einheit am Dispenser angebracht werden.

Solche Systeme können als manuelle oder motorbetriebene Handgeräte ausgebildet sein, an deren Dispenser genau eine Kolben-Zylinder-Einheit anbringbar ist. Es gibt auch Systeme, an deren Dispenser gleichzeitig eine Vielzahl von Kolben-Zylinder-Einheiten angebracht sind, wie z. B. bei einem Pipettierautomaten.

Kolben-Zylinder-Einheiten der in Rede stehenden Art können z. B. als Verdrängereinheiten mit anfügbaren Pipetten oder Spitzen (Tips) oder als Spritzen ausgebildet sein. Sie weisen jeweils einen Zylinder, insbesondere mit einem geraden Hohlzylinder mit einem im Wesentlichen kreisringförmigen Querschnitt und einer dazu senkrechten Axialrichtung, und einen im Zylinder in dessen Axialrichtung verschiebbaren Kolben auf. Durch Verschieben des Kolbens können Fluide in den Zylinder oder in die darin angefügte Spitze aufgenommen und daraus abgegeben werden.

Die Kolben-Zylinder-Einheiten können mittels einer zumindest im Wesentlichen in Axialrichtung des Dispensers verlaufenden Bewegung am Dispenser anbringbar sein. Dies erlaubt eine einfache, nutzerfreundliche, ergonomisch vorteilhafte und weniger fehleranfällige Bedienung des entsprechenden Systems. Dabei kennzeichnet der Ausdruck "Axialrichtung des Dispensers" eine Ausrichtung übereinstimmend mit oder parallel zur Längsachse des Dispensers.

Alternativ können die Kolben-Zylinder-Einheiten mittels einer zumindest im Wesentlichen in Radialrichtung des Dispensers bzw. seitwärts verlaufenden Bewegung am Dispenser anbringbar sein. Dabei kennzeichnet der Ausdruck "Radialrichtung des Dispensers" eine Ausrichtung senkrecht zur Axialrichtung des Dispensers.

Im Fokus der vorliegenden Erfindung stehen Kolben-Zylinder-Einheiten, die nach dem Direktverdrängungsprinzip arbeiten und für das Dispensieren von Flüssigkeiten mit hoher Viskosität und/oder hohem Dampfdruck geeignet sind.

Im Fokus der vorliegenden Erfindung stehen motorbetriebene, elektronische Mehrfachdispenser. Bei solchen Dispensern setzt das Betätigen einer oder mehrerer Taste/n einen Aufnahme- bzw. Aufsaug- oder einen Abgabemechanismus in Gang. Der Kolben einer am Dispenser angebrachten Kolben-Zylinder-Einheit wird mittels eines Motors bewegt, die Volumenaufnahme und/oder -abgabe von einem Mikroprozessor gesteuert. Der Kolben streift die Innenwand des Zylinders der Kolben-Zylinder-Einheit dichtend ab, so dass exakt reproduzierbare Volumenergebnisse erzielt werden.

Für die hier relevanten Systeme ist eine hohe Genauigkeit beim Aufnehmen und Abgeben von Fluidvolumina von Vorteil.

Für die Genauigkeit bzw. für die Richtigkeit und den Variationskoeffizienten bei der Volumenaufnahme bzw. -abgabe mit den Systemen der in Rede stehenden Art ist ein präzises Messen der Länge des Verschiebeweges des Kolbens der Kolben-Zylinder-Einheit im Zylinder wesentlich. Beim Verschieben des Kolbens im Zylinder handelt es sich um eine im Wesentlichen lineare Bewegung in Axialrichtung der Kolben-Zylinder-Einheit.

Aus der EP 0 025 575 A1 ist eine Dosiervorrichtung für kleine Flüssigkeitsmengen im Milli- und Mikroliterbereich bekannt, die ein mit einem Gleichstrommotor angetriebenes Kolben-Zylinder-System, ein Gehäuse, ein optisches digitalinkrementelles Weglängenmesssystem, eine optisch-digitale Messschaltung, und eine Steuer-, Rechen- und Vergleicherschaltung aufweist. Das Weglängenmesssystem hat einen transparenten Impulsstab, der mit undurchsichtigen Markierungen versehen und am Kolben des Kolben-Zylinder-Systems befestigt ist. Die Messschaltung formt den Hub des Kolbens in eine entsprechende Anzahl von digitalen Impulsen um.

Bei der bekannten Dosiervorrichtung ist also bei einer Relativbewegung zwischen dem Kolben und dem Zylinder der zurückgelegte Weg des Kolbens relativ zum Gehäuse des Dispensers inkrementell bestimmbar. Dazu wird die Länge des Verschiebeweges des Kolbens im Zylinder durch Zählen der Impulse und Multiplikation des Zählerstands mit dem Abstand zwischen zwei benachbarten Strichen des Strichgitters (Inkrement) bestimmt. Im Prinzip werden hier also die einzelnen Inkremente aufsummiert, und zwar ausgehend von einem Startpunkt, dessen absolute Position im Gehäuse der Dosiervorrichtung unbekannt ist. Die bekannte Dosiervorrichtung misst also nach Zuschalten der Spannungsversorgung nur Änderungen gegenüber einem unbekannten Startpunkt (Einschalt-Position).

Im Stand der Technik ist ein mikroprozessorgesteuerter Handdispenser, der HandyStep® electronic, zum Dosieren von Flüssigkeitsvolumen aus dem Generalkatalog der Anmelderin (BRAND Gesamtkatalog 900 (Juni 2013)) bekannt. Hier ist eine Spritze radial in den bekannten Dispenser einsetzbar und deren kodierte Größe an einem Kolbenkopf eines Kolbens der Spritze mittels Lichtschranken erfassbar. Der Kolbenkopf wird mit einem Kolbenstellglied gekoppelt. Deren Hubantrieb erfolgt durch einen Elektromotor mit Schraubgetriebe. Der Hub wird durch inkrementelle Drehmessung am Elektromotor und ein Referenzpunkt durch einen gehäusefesten Schalter erfasst. Auch bei diesem Dispenser wird der Weg des Kolbens nur inkrementell und indirekt am Elektromotor erfasst. Zudem sind die Lichtschranken störungsanfällig.

Die US 5 998 218 A offenbart eine mechanische Pipette mit einem elektrischen Aufbau. Die gesamte Zylinderbaugruppe der Pipette ist abnehmbar am Gehäuse befestigt, indem das Zylindergehäuse in einen Buchsenzylinder eingeschraubt wird. Ein vorgesehener Mikroprozessor ist so ausgebildet, dass die Umdrehungszahl des Einstellmechanismus für die Volumenabgabe schrittweise erhöht wird, bevor die tatsächliche Nullposition des Volumenabgabe-Verstellmechanismus erreicht wird. Die Pipette kann durch Betätigung der Auswerferstange und eines Kolbens mit dem Daumen des Benutzers betätigt werden. Das proximale Ende des Kolbens ist am Kolbenadapter befestigt.

Die US 4 567 780 A betrifft eine handbetätigbare Pipette mit einstellbarem Kolbenhub und halbierter elektronischer, digitaler, volumetrischer Digitalanzeige, wobei die Pipette viskose, dichte oder unter hohem Dampfdruck stehende Flüssigkeiten mittels einer leicht austauschbaren Kolbenspitze und Kapillaranordnung aufzieht und abgibt. Das von der Pipette zu übertragende Volumen wird von einer linearen inkrementalen Positionserfassungseinrichtung für die Kolbenanordnung abgeleitet.

Die US 4 821 586 A offenbart eine Pipette, die für den Betrieb mit einem Kolben mit festem Hub vorgesehen ist. Die Pipette umfasst eine Griffanordnung, die einen Schrittmotor enthält, der den Kolben nach unten in eine Ausgangsposition treibt. Der Motor kann eine Gewindewelle umfassen, die durch eng anliegende Führungsbuchsen gestützt wird. Innerhalb des Motors ist eine Mutter, die am Motoranker befestigt ist, auf die Welle aufgebracht. Ein Kunststoffteil, das mit dem Ende der Motorwelle verklebt ist, ist mit einem vorstehenden Stift versehen, der sich durch einen länglichen Schlitz erstreckt. Die Bewegung der Welle und des Kunststoffteils steuert die Bewegung des Kolbens. Die Anzahl der Impulse, die an die Wicklungen vom Motor angelegt werden, bestimmt die Anzahl der Schritte, die Mutter dreht und die Welle bewegt sich, wodurch der Hub vom Kolben und das Volumen der Flüssigkeit bestimmt wird, die in die an der Pipette montierte Einwegspritze eingesaugt wird. Ferner sind Magnete am Außenumfang des Kunststoffteils vorgesehen und arbeiten mit einem Hall-Effekt-Schalter zusammen, um anzuzeigen, dass sich das Kunststoffteil in seiner Ausgangsposition befindet und sich der Kolben in seiner Ausgangsposition befindet und zur Bewegung bereit ist, um Flüssigkeit in eine Einwegspritze anzusaugen oder aufzunehmen.

Die EP 0 576 967 A2 betrifft eine motorgetriebene Knopfpipette, wobei die Verschiebung eines gleitend verschiebbaren Knopfes die Bewegung eines Kolbens mittels eines elektronischen Steuersystems steuert. Der Kolben wird mit Hilfe eines Elektromotors bewegt. Die Bewegung des Motors wird über einen Übertragungsmechanismus, der aus einer Kupplung und einem Zahnradsystem besteht, auf eine gelagerte Führungsschraube übertragen. Die Kupplung hat eine Feder, die das Zahnrad gegen einen Endflansch der Welle drückt. Auf diese Weise gleitet die Kupplung in einen bestimmten Grenzmoment. Am oberen Ende des Kolbens befindet sich eine Führungsmutter, die der Führungsschraube entspricht, und ein Raum im Inneren des Kolbens, so dass sich der Kolben über die Führungsschraube bewegen kann. Wenn die Führungsschraube gedreht wird, bewegt sich der Kolben also je nach Drehrichtung entweder nach oben oder nach unten. Der Bewegung des Kolbens folgt ein System, das über eine rotierende Encoderscheibe und ein Paar Sensoren verfügt. Mit Hilfe des Systems ist es möglich, sowohl die Position als auch die Geschwindigkeit des Kolbens anzuzeigen.

Eine Aufgabe der vorliegenden Erfindung ist es, die bekannte Dosiervorrichtung bzw. das zugehörige System bzw. das entsprechende Verfahren hinsichtlich der Betriebsbereitschaft, der Handhabung der Kolben-Zylinder-Einheit und/oder der Genauigkeit beim Aufnehmen und Abgeben von Fluidvolumina zu verbessern.

Gemäß einem ersten Erfindungsaspekt wird die zuvor geschilderte Aufgabenstellung durch den Dispenser nach Anspruch 1 gelöst. Bevorzugte Ausgestaltungen dieses Erfindungsaspekts sind Gegenstand der diesbezüglichen Unteransprüche.

Es versteht sich von selbst, dass Ausgestaltungen, Ausführungsformen, Vorteile und dergleichen, welche nachfolgend zu Zwecken der Vermeidung von Wiederholungen nur zu einem Erfindungsaspekt angeführt sind, in Bezug auf die übrigen Erfindungsaspekte entsprechend gelten.

Dies vorausgeschickt, wird im Folgenden die vorliegende Erfindung näher beschrieben.

Der erfindungsgemäße Dispenser weist ein Kolbenstellglied auf, das mit dem Kolben der Kolben-Zylinder-Einheit derart lösbar verbindbar ist, dass der Kolben zum Aufnehmen und Abgeben von Fluidvolumina mittels des Kolbenstellglieds bewegbar, nämlich linear verschiebbar ist.

Die Einrichtung zur inkrementellen Wegmessung weist eine Drehmesseinrichtung auf, mittels der die Umdrehungen eines das Kolbenstellglied antreibenden Motors des Dispensers bestimmbar sind und somit der zurückgelegte Weg des Kolbens bei Verstellung des Kolbenstellglieds inkrementell bestimmbar ist. Hier ist die Einrichtung zur inkrementellen Wegmessung also zur indirekten Wegmessung mittels der Umdrehungen des Motors als Hilfsgröße eingerichtet.

Ein Grundgedanke der vorliegenden Erfindung besteht darin, eine inkrementelle Wegmessung einer Relativbewegung zwischen dem Kolben und dem Zylinder mit einer Erfassung der Position des Kolbenstellglieds durch ein mit dem Kolbenstellglied bewegungsmäßig gekoppeltes Positionselement zu kombinieren.

So weist der Dispenser gemäß der vorliegenden Erfindung eine Einrichtung zur Positionsbestimmung auf, die ein erstes Positionselement und ein zweites Positionselement hat. Das erste Positionselement ist starr im bzw. am Gehäuse des Dispensers angeordnet und das zweite Positionselement ist bewegungsmäßig mit dem Kolbenstellglied gekoppelt. Die Einrichtung zur Positionsbestimmung ist zur kontinuierlichen Erfassung der Position des zweiten Positionselements ausgebildet.

Durch die bewegungsmäßige Kopplung des zweiten Positionselements mit dem Kolbenstellglied und die Verbindung des Kolbenstellglieds mit dem Kolben einer an dem Dispenser angebrachten Kolben-Zylinder-Einheit kann die Einrichtung zur Positionsbestimmung die Position des Kolbens direkt und ohne mechanisches Spiel erfassen.

Der erfindungsgemäße Dispenser kann die Position des Kolbens bzw. Kolbenstellglieds unmittelbar nach Zuschalten der Spannungsversorgung, vorzugsweise kontinuierlich aktualisiert, zur Verfügung stellen. Ein Referenzieren ist nicht notwendig. Die erfassten Positionen können jederzeit für weitere Funktionen des Dispensers abgerufen werden.

Mit dem erfindungsgemäßen Dispenser kann z. B. bestimmt werden, wie weit der Kolben in den Zylinder der Kolben-Zylinder-Einheit eingeschoben ist. Sollte der erfindungsgemäße Dispenser beispielsweise feststellen, dass der Kolben nicht maximal in den Zylinder der Kolben-Zylinder-Einheit eingeschoben ist, kann der Dispenser den Kolben mittels des Kolbenstellglieds maximal in den Zylinder einschieben, um eine definierte Ausgangslage z. B. zur Aufnahme eines Fluidvolumens und/oder eines inkrementellen Bestimmens - mittels der Einrichtung zur inkrementellen Wegmessung - des zurückgelegten Weges des Kolbens relativ zum Gehäuse des Dispensers bei einer Relativbewegung zwischen dem Kolben und dem Zylinder zu generieren.

Mit dem erfindungsgemäßen Dispenser kann auch festgestellt werden, ob sich die Position des Kolbens bzw. Kolbenstellglieds in einem ausgeschalteten Zustand des Dispensers geändert hat.

Der erfindungsgemäße Dispenser ermöglicht außerdem eine Justierung der Einrichtung zur inkrementellen Wegmessung und/oder der Einrichtung zur Positionsbestimmung. Ist dem Dispenser beispielsweise eine Referenzposition des Kolbens bzw. Kolbenstellglieds bekannt, so kann der Dispenser anhand eines von der Einrichtung zur inkrementellen Wegmessung bestimmten Weges des Kolbens relativ zum Gehäuse des Dispensers bei einer Relativbewegung zwischen dem Kolben und dem Zylinder ausgehend von dieser Referenzposition eine End- oder Zwischenposition bestimmen und diese End- oder Zwischenposition mit dem Positionswert der Einrichtung zur Positionsbestimmung vergleichen. Sollten die Positionswerte nicht übereinstimmen, kann der Dispenser eine Justierung vornehmen.

Hilfreich ist auch, wenn das zweite Positionselement in einem bekannten Abstand zu einem Referenzpunkt des Kolbens angeordnet ist.

Schließlich ermöglicht der erfindungsgemäße Dispenser eine stetig mögliche Plausibilitätsprüfung der Werte der Einrichtung zur inkrementellen Wegmessung und der Einrichtung zur Positionsbestimmung.

Der erfindungsgemäße Dispenser ermöglicht also auch ein genaues Aufnehmen und Abgeben von Fluidvolumina, insbesondere mit einer hohen Richtigkeit und einem geringen Variationskoeffizienten.

Bei einer ersten bevorzugten Ausführungsform des erfindungsgemäßen Dispensers ist das erste Positionselement ein Positionssensor und das zweite Positionselement ein Positionsmarker. Der Positionssensor ist zur insbesondere kontinuierlichen Erfassung der Position des Positionsmarkers ausgebildet.

Vorzugsweise weist/weisen der Positionssensor eine gedruckte Leiterplatine und/oder der Positionsmarker einen elektrischen Resonator, bevorzugt mit einer Spule und/oder einem Kondensator, auf.

Bei einer zweiten bevorzugten Ausführungsform des erfindungsgemäßen Dispensers ist das erste Positionselement ein Positionsanzeiger, vorzugsweise mit mindestens einem Messkörper, insbesondere zwei Linealen, und das zweite Positionselement ein Positionssensor mit mindestens einem insbesondere optischen Sensor. Der Positionssensor ist zur insbesondere kontinuierlichen Erfassung seiner Position, vorzugsweise zum Ablesen seiner Position am Positionsanzeiger, ausgebildet.

Die nachfolgenden bevorzugten Ausgestaltungen beziehen sich auf beide bevorzugten Ausführungsformen.

Vorzugsweise ist die Einrichtung zur Positionsbestimmung zur optischen, induktiven und/oder kapazitiven, vorzugsweise resonant induktiven, Erfassung der Position des zweiten Positionselements ausgebildet. Dabei ist es bevorzugt, wenn zwischen dem ersten Positionselement und dem zweiten Positionselement kein mechanischer und kein elektrischer Kontakt besteht. Dies ermöglicht ein ungehindertes Bewegen des Kolbenstellglieds im Gehäuse des Dispensers.

Bevorzugt ist die Einrichtung zur Positionsbestimmung zur Bestimmung des zurückgelegten Weges des Kolbens relativ zum ersten Positionselement und/oder zum Gehäuse des Dispensers bei einer Relativbewegung zwischen dem Kolben und dem Zylinder ausgebildet. Dies kann z. B. durch Differenzbildung zweier Positionswerte der Einrichtung zur Positionsbestimmung erfolgen. Somit erfolgt hier quasi eine absolute Wegmessung mittels der Einrichtung zur Positionsbestimmung. Dies ermöglicht u. a. eine Justierung der Wegmessungen beider Einrichtungen und eine Plausibilitätsprüfung der Werte beider Einrichtungen.

Vorzugsweise hat die Einrichtung zur inkrementellen Wegmessung hinsichtlich der Wegmessung eine kleinere Auflösung als die Einrichtung zur Positionsbestimmung (absolute Wegmessung).

Es ist bevorzugt, wenn der Dispenser zum Bestimmen eines variablen Umkehrhubs auf Basis einer Differenzbildung zwischen Messwerten der Einrichtung zur inkrementellen Wegmessung und der Einrichtung zur Positionsbestimmung ausgebildet ist. Alternativ oder zusätzlich kann der Dispenser zum Bestimmen eines nach einem variablen Umkehrhub definierten Startpunktes auf Basis eines Messwertes der Einrichtung zur Positionsbestimmung ausgebildet sein.

Als Umkehrhub wird hier diejenige Wegstrecke des Kolbenstellglieds verstanden, die beim Übergang von der Aufnahme eines Fluidvolumens zur Abgabe eines Fluidvolumens bzw. umgekehrt zurückgelegt wird. Er ist unter anderem mit Spiel und flexibler Deformationen im Antriebszug des das Kolbenstellglied antreibenden Motors begründet. Er hängt z. B. von der Größe und einhergehendem mechanischen Widerstand der am Dispenser angebrachten Kolben-Zylinder-Einheit ab.

Bei gattungsgemäßen Dosiervorrichtungen wird meist ein statischer Umkehrhub eingestellt. Ein statischer Umkehrhub verringert den möglichen nutzbaren Kolbenhub und führt häufig zum ungenauen Aufnehmen und/oder Abgeben von Fluidvolumina.

Der erfindungsgemäße Dispenser kann z. B. durch Vergleich einer inkrementellen Wegmessung (Umdrehungen des Motors) und einer absoluten Wegmessung (Kolbenbewegung zwischen zwei Kolbenpositionen) einen variablen Umkehrhub bestimmen. Damit kann der Kolbenhub besser genutzt und die Genauigkeit erhöht werden.

Ist mittels einer absoluten Wegmessung der Einrichtung zur Positionsbestimmung ein Startpunkt nach einem variablen Umkehrpunkt bestimmt worden, kann ab dem Startpunkt ein zuvor aufgenommenes Fluidvolumen abgegeben werden bzw. wird bei der Aufnahme eines Fluidvolumens die inkrementelle Wegmessung des Kolbenstellglieds begonnen.

Vorzugsweise ist das Kolbenstellglied auf den Kolben, insbesondere einen Kolbenkopf des Kolbens, zu bewegbar, bis ein Anschlag des Kolbenstellglieds an einer Stirnseite des Kolbens anliegt. Diese auch als Blockfahrt bezeichnete Bewegung dient zur Erfassung eines kolbenseitigen Referenzpunkts. Die absolute Position dieses kolbenseitigen Referenzpunkts ist mittels der Einrichtung zur Positionsbestimmung erfassbar.

Vorteilhafterweise weist der Dispenser ein Erfassungselement auf, das zumindest teilweise in eine axial ausgerichtete Aussparung, insbesondere Nut, in der Stirnseite des Kolbens in Axialrichtung einführbar ist. Das zweite Positionselement bzw. der Positionsmarker ist bewegungsmäßig mit dem Erfassungselement gekoppelt. Mittels der Einrichtung zur Positionsbestimmung ist die Tiefe der Aussparung bestimmbar.

Die axial ausgerichtete Aussparung stellt einen Informationsträgerabschnitt dar. Die Tiefe der Aussparung spezifiziert den Typ der Kolben-Zylinder-Einheit zumindest teilweise. Dabei bezeichnet der Begriff "Typ" z. B. einen Zweck, einen Zustand und/oder eine Eigenschaft der Kolben-Zylinder-Einheit, wie z. B. das maximal aufnehmbare und/oder abgebbare Fluidvolumen.

Der Ausdruck "axial ausgerichtete Aussparung" ist dabei so zu verstehen, dass diese Aussparung in axialer Richtung der Kolben-Zylinder-Einheit zugänglich und/oder deren Information in axialer Richtung der Kolben-Zylinder-Einheit erfassbar ist. Die axial ausgerichtete Aussparung hat in Axialrichtung eine geometrische Ausdehnung, mittels der die erfassbare Information kodiert ist. Außerdem hat die axial ausgerichtete Aussparung eine geometrische Ausdehnung in Radialrichtung und in Umfangsrichtung. Die axial ausgerichtete Aussparung kann als Nut, also nicht vollständig von Kolbenmaterial umgeben, ausgeführt sein. Eine solche Ausgestaltung erleichtert ein Säubern der Aussparung und bietet eine einfache Kontrollmöglichkeit.

Der kolbenseitige Referenzpunkt markiert insbesondere den Ausgangspunkt einer Bestimmung der Tiefe der axial ausgerichteten Aussparung des Kolbens.

Vorzugsweise ist das Erfassungselement stößelartig und/oder stiftförmig ausgebildet und/oder federbelastet, insbesondere gegen die Einlegerichtung der Kolben-Zylinder-Einheit elastisch vorgespannt. Besonders bevorzugt ist, wenn der Dispenser ein Verriegelungselement aufweist, mit dem das Erfassungselement in eine Freigabeposition bewegbar und dort haltbar bzw. blockierbar ist. Sobald das Verriegelungselement das Erfassungselement nicht mehr blockiert, wird das federbelastete Erfassungselement auf den Kolben zu bewegt und schließlich in die in Axialrichtung verlaufende Aussparung des Kolbens gedrückt.

Vorzugsweise hat der Dispenser Mittel, insbesondere eine Lichtschranke, zur mechanischen, elektronischen, induktiven und/oder optischen Erkennung des Anbringens der Kolben-Zylinder-Einheit am Dispenser.

Bevorzugt ist der erfindungsgemäße Dispenser ein völlig autonomes, von Hand haltbares und motorbetriebenes Gerät, das standortunabhängig sämtliche Komponenten in einem Gehäuse vereinigt. Hierzu zählen wie üblich ein Antrieb, vorzugsweise ein Motorantrieb, eine Getriebeeinrichtung, die die Drehbewegung des Motors in eine Längsbewegung des Kolbenstellglieds umwandelt, eine Elektronik, eine Stromversorgung und natürlich eine Ankoppeleinrichtung zur Verbindung des Kolbens der Kolben-Zylinder-Einheit mit dem Kolbenstellglied.

Gemäß einem zweiten Erfindungsaspekt wird die zuvor geschilderte Aufgabenstellung durch ein System zum Aufnehmen und Abgeben von Fluidvolumina gemäß Anspruch 8 gelöst.

Das System gemäß dem zweiten Erfindungsaspekt weist eine als Austauschteil ausgebildete Kolben-Zylinder-Einheit und einen wie zuvor beschriebenen Dispenser auf. Die Kolben-Zylinder-Einheit ist am Dispenser lösbar angebracht und weist einen Kolben und einen am Dispenser fixierten Zylinder auf. Der Dispenser hat ein Kolbenstellglied, das mit dem Kolben derart lösbar verbunden ist, dass der Kolben zum Aufnehmen und Abgeben von Fluidvolumina mittels des Kolbenstellglieds bewegbar, nämlich linear verschiebbar ist.

Die zuvor geschilderte Aufgabenstellung wird auch durch ein Verfahren zum Aufnehmen und Abgeben von Fluidvolumina gelöst, wie es nachfolgend erläutert ist.

Das Verfahren dient zum Aufnehmen und Abgeben von einstellbaren Fluidvolumina mittels eines Dispensers, an dem eine als Austauschteil ausgebildete Kolben-Zylinder-Einheit lösbar anbringbar ist. Die Kolben-Zylinder-Einheit weist einen Kolben und einen Zylinder auf. Der Dispenser weist ein Gehäuse und ein Kolbenstellglied auf. Insbesondere ist der Dispenser wie weiter oben beschrieben ausgebildet.

Das Verfahren umfasst die folgenden Verfahrensschritte:
a) Lösbares Anbringen der Kolben-Zylinder-Einheit am Dispenser, vorzugsweise mittels einer zumindest im Wesentlichen in Axialrichtung des Dispensers verlaufenden Bewegung;
b) Lösbares Verbinden des Kolbens mit dem Kolbenstellglied, so dass der Kolben zum Aufnehmen und Abgeben von Fluidvolumina mittels des Kolbenstellglieds linear verschiebbar ist;
c) Erzeugen einer Relativbewegung zwischen dem Kolben und dem Zylinder mittels des Kolbenstellglieds, indem das Kolbenstellglied den Kolben linear verschiebt;
d) Inkrementelles Bestimmen des zurückgelegten Wegs des Kolbens relativ zum Gehäuse des Dispensers, wobei mittels einer Drehmesseinrichtung des Dispensers die Umdrehungen eines das Kolbenstellglied antreibenden Motors des Dispensers bestimmt werden und somit die Länge des linearen Verschiebeweges des Kolbens im Zylinder bei Verstellung des Kolbenstellglieds inkrementell bestimmt wird, während das Kolbenstellglied durch den Motor bewegt wird; und
e) Kontinuierliches Erfassen der absoluten Wegposition eines Positionselements des Dispensers, das bewegungsmäßig mit dem Kolbenstellglied gekoppelt ist.

Durch Schritt b) des Verfahrens wird eine Kopplung zwischen dem Kolbenstellglied und dem Kolben erreicht.

Vorzugsweise wird in Schritt a) der Zylinder der Kolben-Zylinder-Einheit am Dispenser fixiert.

Es ist bevorzugt, wenn die Fertigstellung eines erfolgreichen Anbringens der Kolben-Zylinder-Einheit am Dispenser mechanisch, elektronisch, induktiv und/oder optisch erfasst wird, vorzugsweise mittels einer Lichtschranke.

Vorteilhafterweise wird anhand erfasster Positionen des Positionselements eine Richtungsumkehr der Relativbewegung zwischen dem Kolben und dem Zylinder erfasst. Ein Startpunkt für das Erfassen einer weiteren Relativbewegung kann bestimmt werden.

Bevorzugt wird mindestens ein inkrementell bestimmter zurückgelegter Weg des Kolbens in Bezug gesetzt zu mindestens einer erfassten Position des Positionselements. Auf dieser Basis kann ein variabler Umkehrhub bestimmt und/oder die Relativbewegung zwischen dem Kolben und dem Zylinder erfasst, eine Justierung der Wegbestimmung und/oder der Positionserfassung vorgenommen und/oder Plausibilitätsprüfungen hinsichtlich der bestimmten Wegstrecken und/oder der erfassten Positionen durchgeführt werden.

Bei einer bevorzugten Ausführungsform des Verfahrens wird ein kolbenseitiger Referenzpunkt erfasst, indem das Kolbenstellglied des Dispensers auf den Kolben zu bewegt wird, bis ein Anschlag des Kolbenstellglieds an einer Stirnseite des Kolbens anliegt. Dabei kann der vom Kolbenstellglied zurückgelegte Weg bestimmt werden, insbesondere mittels der Einrichtung zur inkrementellen Wegmessung.

Vorzugsweise wird ein Erfassungselement des Dispensers zumindest teilweise in eine axial ausgerichtete Aussparung in einer Stirnseite des Kolbens in Axialrichtung eingeführt und die Tiefe dieser Aussparung anhand erfasster Positionen des zweiten Positionselements oder Positionsmarkers bestimmt. Der bestimmte Tiefenwert kann zur Identifizierung des Typs der Kolben-Zylinder-Einheit verwendet werden.

Die Erfindung wird nachfolgend anhand der Beschreibung bevorzugter Ausführungsbeispiele, zum Teil mit Bezugnahmen auf die Zeichnung, näher erläutert. Die oben beschriebenen und/oder in den Ansprüchen und/oder in der nachfolgenden Beschreibung offenbarten Merkmale können bedarfsweise miteinander kombiniert aber auch unabhängig voneinander realisiert werden, auch wenn dies nicht im Einzelnen ausdrücklich beschrieben ist.

In der Zeichnung zeigt
- Fig. 1: schematisch in einer perspektivischen Ansicht eine bevorzugte Ausführungsform eines erfindungsgemäßen Dispensers,
- Fig. 2: schematisch in einer perspektivischen Ansicht eine bevorzugte Ausführungsform eines erfindungsgemäßen Systems mit dem Dispenser von Fig. 1 und einer in den Dispenser eingelegten Kolben-Zylinder-Einheit, wobei eine vereinfachte Darstellung unter Weglassung von Teilen des Dispensers gewählt wurde, und
- Fig. 3: schematisch in einer perspektivischen Ansicht einen Längsschnitt durch das System von Fig. 2.

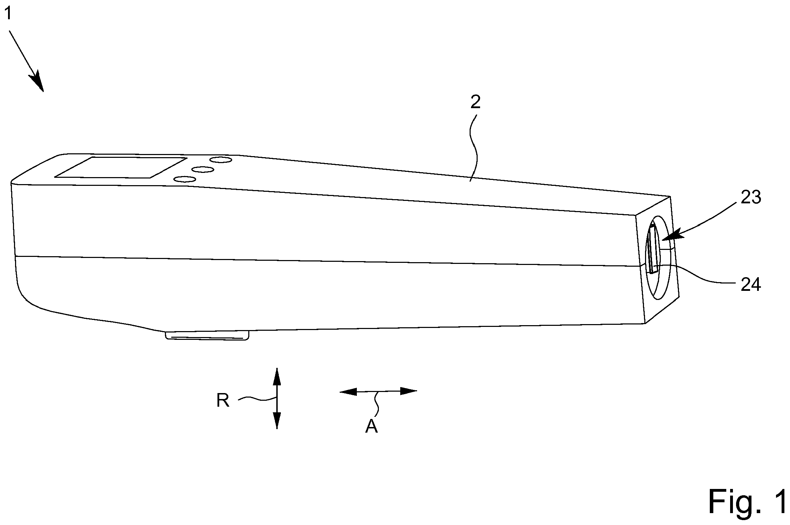

Fig. 1 zeigt schematisch in einer perspektivischen Ansicht eine bevorzugte Ausführungsform eines erfindungsgemäßen Dispensers 1 zum Aufnehmen und Abgeben von Fluidvolumina. Der Dispenser 1 hat ein Gehäuse 2 mit einer Öffnung 23 und eine hinter der Öffnung 23 innerhalb des Gehäuses 2 angeordnete Fixiereinrichtung 24 für die Kolben-Zylinder-Einheit 3.

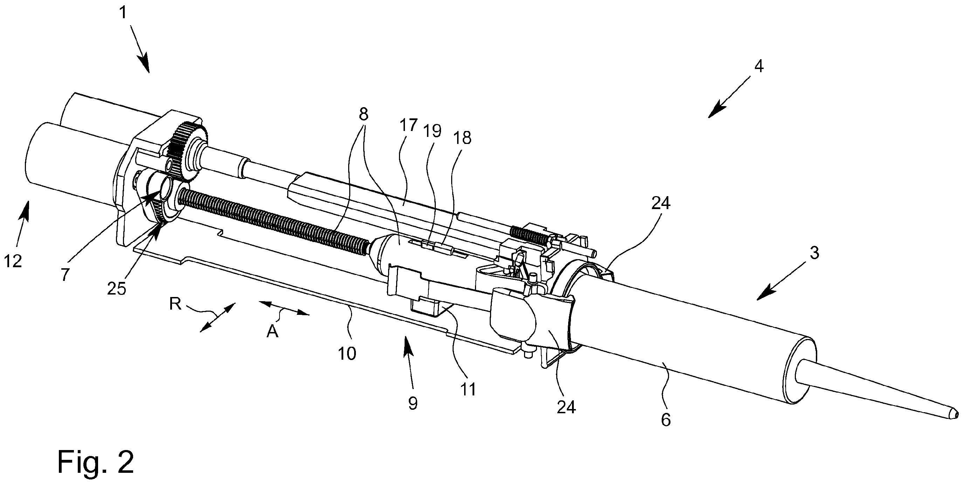

Fig. 2 zeigt schematisch in einer perspektivischen Ansicht den Dispenser 1 aus Fig. 1 und einer in den Dispenser 1 eingelegten, von der Fixiereinrichtung 24 gehaltenen Kolben-Zylinder-Einheit 3 als Teile einer bevorzugten Ausführungsform eines erfindungsgemäßen Systems 4 zum Aufnehmen und Abgeben von Fluidvolumina. Dabei ist der Dispenser 1 nur teilweise dargestellt. Fig. 2 beschränkt sich nämlich auf die Darstellung derjenigen Komponenten, die zur Erläuterung der vorliegenden Erfindung erforderlich sind.

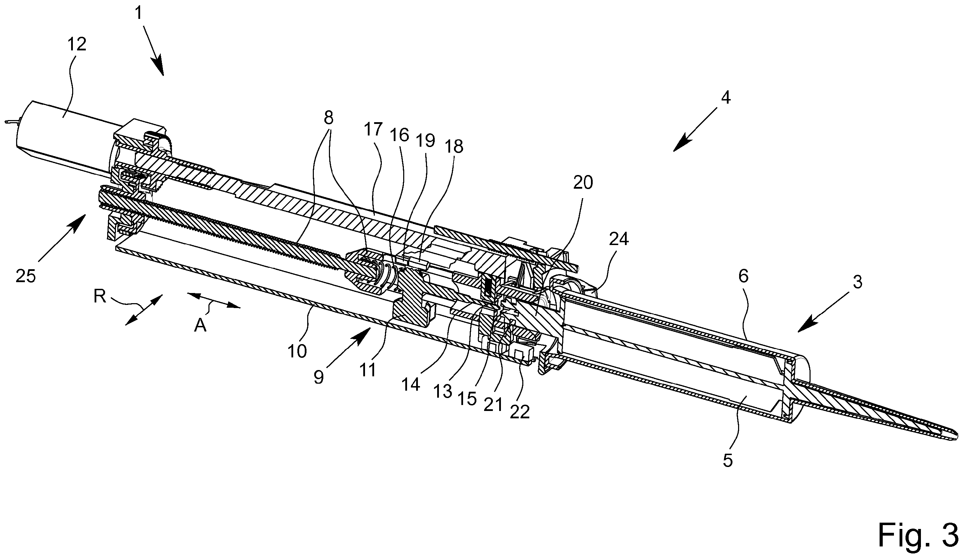

Fig. 3 zeigt schematisch in einer perspektivischen Ansicht einen Längsschnitt durch das System 4 von Fig. 2.

Der Dispenser 1 und die Kolben-Zylinder-Einheit 3 haben eine in Fig. 2 dargestellte Axialrichtung A und eine zur Axialrichtung A senkrechte und durch die Längsachse des Dispensers 1 bzw. der Kolben-Zylinder-Einheit 3 verlaufende Radialrichtung R. Die Kolben-Zylinder-Einheit 3 ist als Austauschteil ausgebildet. Sie kann in Form einer Spritze ausgebildet sein und in verschiedenen Größen mit unterschiedlichem Aufnahmevolumen vorliegen. Sie weist einen abgedichteten Kolben 5 auf, der in einem Zylinder 6 der Kolben-Zylinder-Einheit 3 zum Zwecke des Aufnehmens bzw. Ansaugens und Abgebens bzw. Ausstoßens einer zu pipettierenden oder dosierenden Flüssigkeit bewegbar ist. In Fig. 2 ist der Kolben 5 nicht sichtbar.

Im eingelegten Zustand der Kolben-Zylinder-Einheit 3 liegt der Zylinder 6 an einer nicht dargestellten Wippe des Dispensers 1 an. Hiermit und mit Hilfe der Fixiereinrichtung 24 ist der Zylinder 6 am Dispenser 1 bzw. an dem System 4 fixiert, so dass zum Aufnehmen bzw. Ansaugen und Abgeben bzw. Ausstoßen von Flüssigkeit hinsichtlich der Kolben-Zylinder-Einheit 3 nur der Kolben 5 im Zylinder 6 bewegbar ist.

Der Dispenser 1 hat eine Einrichtung zur inkrementellen Wegmessung 7, die so ausgebildet ist, dass bei einer Relativbewegung zwischen dem Kolben 5 und dem Zylinder 6 der zurückgelegte Weg des Kolbens 5 relativ zum Gehäuse 2 des Dispensers 1 inkrementell bestimmbar ist.
Der Dispenser 1 bzw. das System 4 weist ein Kolbenstellglied 8 auf, das mit dem Kolben 5 der Kolben-Zylinder-Einheit 3 derart lösbar verbindbar ist, dass der Kolben 5 zum Aufnehmen und/oder Abgeben von Fluidvolumina mittels des Kolbenstellglieds 8 bewegbar ist.

Der Dispenser 1 bzw. das System 4 weist eine Einrichtung zur Positionsbestimmung 9 auf, die ein erstes Positionselement 10 und ein zweites Positionselement 11 hat. Das erste Positionselement 10 ist starr im bzw. am Gehäuse 2 des Dispensers 1 angeordnet. Das zweite Positionselement 11 ist bewegungsmäßig mit dem Kolbenstellglied 8 gekoppelt, und zwar dadurch, dass das zweite Positionselement 11 am Kolbenstellglied 8 angeordnet ist. Die Einrichtung zur Positionsbestimmung 9 ist zur Erfassung der Position des zweiten Positionselements 11 ausgebildet.

Bei der dargestellten und bevorzugten Ausführungsform kann der Dispenser 1 die jeweilige Position des zweiten Positionselements 11 unmittelbar nach Zuschalten der Spannungsversorgung zur Verfügung stellen. Die jeweilige Position wird kontinuierlich erfasst und kann jederzeit von einer weiteren Elektronik des Dispensers 1 abgerufen werden.

Bei der dargestellten und bevorzugten Ausführungsform ist das erste Positionselement 10 ein Positionssensor und das zweite Positionselement 11 ein Positionsmarker. Der Positionssensor 10 ist zur Erfassung der Position des Positionsmarkers 11 ausgebildet.

Die Einrichtung zur Positionsbestimmung 9 ist hier zur resonant induktiven Erfassung der Position des Positionsmarkers 11 ausgebildet. Zwischen dem Positionssensor 10 und dem Positionsmarker 11 besteht kein mechanischer und kein elektrischer Kontakt.

Der Positionssensor 10 weist hier eine gedruckte Leiterplatine auf. Der Positionsmarker 11 hat einen elektrischen Resonator mit mehreren Spulen und einen Kondensator. Dabei wird eine der Spulen verwendet, um dem Resonator Energie zur Verfügung zu stellen. Die Spulen sind so angeordnet, dass bei einer Bewegung des Positionsmarkers 11 eine Sinus/Kosinus-Variation des Kopplungsfaktors erzeugt wird. Die Berechnung der jeweiligen Position des Positionsmarkers 11 entspricht der Berechnung eines Phasenwinkels und wird anhand einer inversen Vier-Quadranten-Tangente durchgeführt.

Die Einrichtung zur Positionsbestimmung 9 ermöglicht eine einfache, robuste und kostengünstige Erfassung der Position des Positionsmarkers 11.

Bei der dargestellten und bevorzugten Ausführungsform wird das Kolbenstellglied 8 über eine aus dem Stand der Technik bekannte Getriebeeinrichtung 25 von einem Motor 12 angetrieben. Die Einrichtung zur inkrementellen Wegmessung 7 weist eine Drehmesseinrichtung auf, mittels der die Umdrehungen des Motors 12 des Dispensers 1 bestimmbar sind. Auf Basis der bestimmten Umdrehungen und eines Umrechnungsfaktors der Getriebeeinrichtung 25 ist der zurückgelegte Weg des Kolbens 5 bei Verstellung des Kolbenstellglieds 8 inkrementell bestimmbar.

Bei der dargestellten und bevorzugten Ausführungsform ist die Einrichtung zur Positionsbestimmung 9 zur Bestimmung des zurückgelegten Weges des Kolbens 5 relativ zum Positionssensor 10 und zum Gehäuse 2 bei einer Relativbewegung zwischen dem Kolben 5 und dem Zylinder 6 ausgebildet. Insbesondere ist die Einrichtung zur Positionsbestimmung 9 zur Differenzbildung zweier erfasster Positionswerte ausgebildet. Somit kann die Einrichtung zur Positionsbestimmung 9 eine absolute Wegmessung vornehmen. Dabei hat die Einrichtung zur inkrementellen Wegmessung 7 hinsichtlich der Wegmessung eine kleinere Auflösung als die Einrichtung zur Positionsbestimmung 9.

Bei der dargestellten und bevorzugten Ausführungsform ist der Dispenser 1 zum Bestimmen eines variablen Umkehrhubs auf Basis einer Differenzbildung zwischen Messwerten der Einrichtung zur inkrementellen Wegmessung 7 und der Einrichtung zur Positionsbestimmung 9 ausgebildet. Zusätzlich ist der Dispenser 1 zum Bestimmen eines nach einem variablen Umkehrhub definierten Startpunktes auf Basis eines Messwertes der Einrichtung zur Positionsbestimmung 9 ausgebildet. Ab dem so bestimmten Startpunkt kann ein zuvor aufgenommenes Fluidvolumen abgegeben werden bzw. wird bei der Aufnahme eines Fluidvolumens jeweils die inkrementelle Wegmessung des Kolbenstellglieds 8 begonnen.

Bei der dargestellten und bevorzugten Ausführungsform ist das Kolbenstellglied 8 zur Erfassung eines kolbenseitigen Referenzpunkts auf den Kolben 5 zu bewegbar, bis ein Anschlag 13 des Kolbenstellglieds 8 an einer Stirnseite des Kolbens 5 anliegt. Die absolute Position dieses kolbenseitigen Referenzpunkts ist mittels der Einrichtung zur Positionsbestimmung 9 und/oder mittels der Einrichtung zur inkrementellen Wegmessung 7 erfassbar.

Bei der dargestellten und bevorzugten Ausführungsform weist der Dispenser 1 ein Erfassungselement 14 auf, das zumindest teilweise in eine axial ausgerichtete Aussparung 15 in einer Stirnseite des Kolbens 5 in Axialrichtung A einführbar ist. Der Positionsmarker 11 ist bewegungsmäßig mit dem Erfassungselement 14 gekoppelt. Mittels der Einrichtung zur Positionsbestimmung 9 ist die Tiefe der Aussparung 15 bestimmbar. Auf Basis der so bestimmten Tiefe der Aussparung 15 kann der Typ der Kolben-Zylinder-Einheit 3 mit seinen individuellen Dimensionen und Anforderungen bei der Verwendung mit dem Dispenser 1 zumindest teilweise identifiziert werden.

Der zuvor bestimmte kolbenseitige Referenzpunkt markiert den Ausgangspunkt einer Bestimmung der Tiefe der Aussparung 15.

Das Erfassungselement 14 ist hier mittels einer Feder 16 gegen die Einlegerichtung der Kolben-Zylinder-Einheit 3 elastisch vorgespannt. Der Dispenser 1 weist ein Verriegelungselement 17 auf, mit dem das Erfassungselement 14 in eine Freigabeposition bewegbar und dort haltbar ist. In der Freigabeposition hält das Verriegelungselement 17 das Erfassungselement 14, indem ein Mitnehmer 18 des Verriegelungselements 17 an einem Fortsatz 19 des Erfassungselements 14 der Kraft der Feder 16 entgegenwirkt und eine Bewegung des Erfassungselements 14 in Richtung der Federkraft (also in Axialrichtung A auf die Kolben-Zylinder-Einheit 3 zu) blockiert.

Das Verriegelungselement 17 ist in Axialrichtung A im Dispenser 1 und relativ zum Gehäuse 2 und zum Kolbenstellglied 8 verschiebbar. Durch Verschieben des Verriegelungselements 17 in Richtung der Kolben-Zylinder-Einheit 3 wird eine Bewegung des Erfassungselements 14 nicht mehr blockiert, so dass das Erfassungselement 14 durch die Feder 16 auf den Kolben 5 zu und dann in die axial ausgerichtete Aussparung 15 gedrückt wird, bis das Erfassungselement 14 auf das Ende der Aussparung 15 trifft und dort blockiert wird.

Die axial ausgerichtete Aussparung 15 ist hier in Axialrichtung A nach oben hin, in Fig. 3 also nach links hin, offen. Die Aussparung 15 ist hier in Form einer Nut ausgebildet. Es handelt sich also nicht um eine Bohrung, die in radialer Richtung R umschlossen ist. Vielmehr ist die Aussparung 15 in radialer Richtung R teilweise nach außen hin offen, und zwar entlang der gesamten Tiefe der Aussparung 15. Alternativ kann die axial ausgerichtete Aussparung 15 als ein Sackloch ausgebildet sein.

Bei der dargestellten und bevorzugten Ausführungsform kann die Tiefe der axial ausgerichteten Aussparung 15 einen von mehreren diskreten Werten annehmen, z. B. einen von acht möglichen Tiefenwerten. Die diskreten Tiefenwerte weisen einen Mindestabstand auf, vorzugsweise ca. 2 mm. Bei einer Tiefe von 0 mm handelt es sich nicht um eine Aussparung im Sinne der vorliegenden Erfindung.

Bei der dargestellten und bevorzugten Ausführungsform hat der Kolben 5, insbesondere ein Kolbenkopf 20 des Kolbens 5, einen den Typ der Kolben-Zylinder-Einheit 3 teilweise spezifizierenden, radial ausgerichteten Informationsträgerabschnitt 21. Dabei ist der radial ausgerichtete Informationsträgerabschnitt 21 durch eine in Radialrichtung R verlaufende und in Umfangsrichtung der Kolben-Zylinder-Einheit 3 umlaufende Aussparung 21 am Kolbenkopf 20 gebildet. In Fig. 3 ist zu erkennen, dass die radial ausgerichtete Aussparung 21 dadurch gebildet ist, dass der Kolbenkopf 20 in diesem Abschnitt einen kleineren Durchmesser hat.

Bei der dargestellten und bevorzugten Ausführungsform spezifizieren die Tiefe der axial ausgerichteten Aussparung 15 und die Tiefe der radial ausgerichteten Aussparung 21 den Typ der Kolben-Zylinder-Einheit 3.

Bei der dargestellten und bevorzugten Ausführungsform hat der Dispenser 1 eine Sensoreinrichtung zur Erkennung des Anbringens der Kolben-Zylinder-Einheit 3 am Dispenser 1. Als Sensoreinrichtung ist hier eine Lichtschranke 22 vorgesehen. Nachdem ein erfolgreiches Anbringen einer Kolben-Zylinder-Einheit 3 erkannt wurde, können Fluide in den Zylinder 6 aufgenommen bzw. aus dem Zylinder 6 abgegeben werden.

Der Dispenser 1 hat weitere, nicht dargestellte Einrichtungen, u. a. eine elektronische Steuereinrichtung, eine Getriebeeinrichtung, eine Stromversorgungseinrichtung, eine Ankoppeleinrichtung zum Verbinden des Kolbens 5 mit dem Kolbenstellglied 8, eine Anzeigeeinrichtung und eine Eingabeeinrichtung.

Im Folgenden wird ein bevorzugter Ablauf eines Verfahrens zum Aufnehmen und Abgeben von Fluidvolumina geschildert.

Zunächst wird die Kolben-Zylinder-Einheit 3 in den Dispenser 1 mittels einer zumindest im Wesentlichen in Axialrichtung A verlaufenden Bewegung lösbar eingelegt. Der Zylinder 6 der Kolben-Zylinder-Einheit 3 wird fixiert.

Ist die Kolben-Zylinder-Einheit 3 erfolgreich in den Dispenser 1 eingelegt worden, wird dies mittels der Sensoreinrichtung, hier der Lichtschranke 22, erfasst.

Danach wird der kolbenseitige Referenzpunkt durch Erzeugen einer Relativbewegung zwischen dem Kolben 5 und dem Kolbenstellglied 8 erfasst. Dabei wird das Kolbenstellglied 8 auf den Kolben 5 zu bewegt, bis der Anschlag 13 des Kolbenstellglieds 8 an einer Stirnseite des Kolbens 5 anliegt (Blockfahrt). Die Länge der Relativbewegung wird mittels der Einrichtung zur inkrementellen Wegmessung 7 oder der Einrichtung zur Positionsbestimmung 9 bestimmt.
Es folgt die Erfassung des kolbenseitigen Referenzpunktes als absolute Wegposition mittels der Einrichtung zur Positionsbestimmung 9, die bewegungsmäßig mit dem Kolbenstellglied 8 gekoppelt ist. Der Referenzpunkt stellt sozusagen eine Weg-Kalibrierung dar, die das Verfahren zumindest weitgehend von Unterschieden in den Maßtoleranzen der Teile der Kolben-Zylinder-Einheit 3 unabhängig macht. Denn die Bestimmung des Referenzpunktes ermöglicht, einen Ausgangspunkt für das Auslesen der Information der Aussparungen 15, 21 festzulegen.

Bei der bevorzugten Ausführungsform des Verfahrens wird nach der Blockfahrt das Erfassungselement 14 des Dispensers 1 zumindest teilweise in die axial ausgerichtete Aussparung 15 in Axialrichtung A eingeführt und die Tiefe dieser Aussparung 15 anhand erfasster Positionen des Positionsmarkers 11 bestimmt. Der bestimmte Tiefenwert wird zur teilweisen Identifizierung des Typs der Kolben-Zylinder-Einheit 3 verwendet.

Danach, gleichzeitig oder davor wird der Kolbenkopf 20 der Kolben-Zylinder-Einheit 3 mit dem Kolbenstellglied 8 des Dispensers 1 lösbar verbunden. Auf diese Weise wird eine Kopplung zwischen dem Kolbenstellglied 8 und dem Kolben 5 erreicht.

Danach wird eine Relativbewegung zwischen dem Kolben 5 und dem Zylinder 6 erzeugt, indem das Kolbenstellglied 8 den Kolben 5 bewegt, insbesondere linear verschiebt. Dabei wird das Kolbenstellglied 8 durch den Motor 12 angetrieben. Der bei dieser Relativbewegung vom Kolbenstellglied 8 zurückgelegte Weg entspricht dem dabei vom Kolben 5 zurückgelegten Weg.

Der bei dieser Relativbewegung vom Kolben 5 zurückgelegte Weg relativ zum Gehäuse 2 des Dispensers 1 wird inkrementell bestimmt mittels der Einrichtung zur inkrementellen Wegmessung 7. Dabei werden die Umdrehungen des das Kolbenstellglied 8 antreibenden Motors 12 bestimmt.

Außerdem wird kontinuierlich die absolute Wegposition des Positionsmarkers 11 des Dispensers 1 mittels der Einrichtung zur Positionsbestimmung 9 erfasst. Durch Differenzbildung zweier absoluter Wegpositionen werden absolute Wegstrecken berechnet.

Findet bei der Relativbewegung zwischen dem Kolben 5 und dem Zylinder 6 eine Richtungsumkehr statt, z. B. beim Wechsel vom Aufnehmen zur Abgabe eines Fluidvolumens oder umgekehrt, wird anhand der kontinuierlichen Erfassung der absoluten Wegpositionen des Positionsmarkers 11 diese Richtungsumkehr direkt am Kolbenstellglied 8 und dem daran gekoppelten Kolben 5 erfasst. Der Hub des antreibenden Motors 12 bei der Richtungsumkehr, ohne dass sich das Kolbenstellglied 8 bewegt und eine Relativbewegung zwischen dem Kolben 5 und Zylinder 6 erzeugt, ist durch unterschiedliche Antriebskräfte, mechanisches Spiel und Verschleiß variabel. Beides, der Umkehrhub und die Relativbewegung können unabhängig erfasst werden. Außerdem wird ein Startpunkt nach der Richtungsumkehr für das Erfassen einer weiteren Relativbewegung bestimmt. Ab dem so bestimmten Startpunkt wird der vom Kolben 5 zurückgelegte Weg relativ zum Gehäuse 2 mittels der Einrichtung zur inkrementellen Wegmessung 7 inkrementell bestimmt.

Bei der bevorzugten Ausführungsform des erfindungsgemäßen Verfahrens werden die Werte inkrementeller Wegmessungen in Bezug gesetzt zu erfassten Positionen des Positionsmarkers 11, insbesondere absoluten Wegmessungen anhand der erfassten Positionen des Positionsmarkers 11. Auf dieser Basis wird eine Justierung der Wegmessung und der Positionserfassung vorgenommen. Dazu werden die in Bezug gesetzten Werte miteinander verglichen und etwaige Abweichungen zur Justierung verwendet. Auf diese Weise werden auch Plausibilitätsprüfungen hinsichtlich der bestimmten Wegstrecken und der erfassten Positionen durchgeführt. Nicht plausible Werte können beispielsweise verworfen werden.

### Bezugszeichenliste:

- 1: Dispenser
- 2: Gehäuse von 1
- 3: Kolben-Zylinder-Einheit
- 4: System
- 5: Kolben von 3
- 6: Zylinder von 3
- 7: Einrichtung zur inkrementellen Wegmessung von 1
- 8: Kolbenstellglied von 1
- 9: Einrichtung zur Positionsbestimmung
- 10: erstes Positionselement von 9
- 11: zweites Positionselement von 9
- 12: Motor von 1
- 13: Anschlag von 8
- 14: Erfassungselement von 1
- 15: axial ausgerichtete Aussparung von 5
- 16: Feder von 8
- 17: Verriegelungselement von 1
- 18: Mitnehmer von 17
- 19: Fortsatz von 14
- 20: Kolbenkopf
- 21: radial ausgerichtete Aussparung von 5
- 22: Lichtschranke von 1
- 23: Öffnung von 2
- 24: Fixiereinrichtung von 1
- 25: Getriebeeinrichtung von 1
- A: Axialrichtung
- R: Radialrichtung

## Patentansprüche

1. Dispenser zum Aufnehmen und Abgeben von Fluidvolumina, an dem eine als Austauschteil ausgebildete Kolben-Zylinder-Einheit (3) mit einem Kolben (5) und einem Zylinder (6) lösbar anbringbar ist,
wobei der Dispenser (1) ein Gehäuse (2) und eine Einrichtung zur inkrementellen Wegmessung (7) aufweist, die so ausgebildet ist, dass bei einer Relativbewegung zwischen dem Kolben (5) und dem Zylinder (6) der zurückgelegte Weg des Kolbens (5) relativ zum Gehäuse (2) des Dispensers (1) inkrementell bestimmbar ist,
wobei der Dispenser (1) ein Kolbenstellglied (8) aufweist, das mit dem Kolben (5) derart lösbar verbindbar ist, dass der Kolben (5) zum Aufnehmen und Abgeben von Fluidvolumina mittels des Kolbenstellglieds (8) linear verschiebbar ist,
wobei die Einrichtung zur inkrementellen Wegmessung (7) eine Drehmesseinrichtung aufweist, mittels der die Umdrehungen eines das Kolbenstellglied (8) antreibenden Motors (12) des Dispensers (1) bestimmbar sind und somit die Länge des linearen Verschiebeweges des Kolbens (5) im Zylinder (6) bei Verstellung des Kolbenstellglieds (8) inkrementell bestimmbar ist,
**dadurch gekennzeichnet, dass**
der Dispenser (1) eine Einrichtung zur Positionsbestimmung (9) aufweist, die ein erstes Positionselement (10) und ein zweites Positionselement (11) hat,
das erste Positionselement (10) starr im bzw. am Gehäuse (2) des Dispensers (1) angeordnet und das zweite Positionselement (11) bewegungsmäßig mit dem Kolbenstellglied (8) gekoppelt ist,
die Einrichtung zur Positionsbestimmung (9) zur kontinuierlichen Erfassung der absoluten Wegposition des zweiten Positionselements (11) ausgebildet ist.

2. Dispenser nach Anspruch 1, **dadurch gekennzeichnet, dass** das erste Positionselement (10) ein Positionssensor und das zweite Positionselement (11) ein Positionsmarker ist und der Positionssensor (10) zur kontinuierlichen Erfassung der absoluten Wegposition des Positionsmarkers (11) ausgebildet ist, vorzugsweise wobei der Positionssensor (10) eine gedruckte Leiterplatine aufweist.

3. Dispenser nach Anspruch 2, **dadurch gekennzeichnet, dass** der Positionsmarker (11) einen elektrischen Resonator, vorzugsweise mit einer Spule und/oder einem Kondensator, aufweist.

4. Dispenser nach Anspruch 1, **dadurch gekennzeichnet, dass** das erste Positionselement (10) ein Positionsanzeiger mit mindestens einem Messkörper, und das zweite Positionselement (11) ein Positionssensor mit mindestens einem Sensor ist und der Positionssensor (11) zur kontinuierlichen Erfassung seiner absoluten Wegposition, nämlich zum Ablesen seiner Position am Positionsanzeiger (10) ausgebildet ist.

5. Dispenser nach einem der voranstehenden Ansprüche, **dadurch gekennzeichnet, dass** die Einrichtung zur Positionsbestimmung (9) zur optischen, induktiven und/oder kapazitiven, vorzugsweise resonant induktiven, Erfassung der Position des zweiten Positionselements (11) ausgebildet ist.

6. Dispenser nach einem der voranstehenden Ansprüche, **dadurch gekennzeichnet, dass** die Einrichtung zur Positionsbestimmung (9) zur Erfassung der Position des zweiten Positionselements (11) ohne mechanischen und ohne elektrischen Kontakt zwischen dem ersten Positionselement (10) und dem zweiten Positionselement (11) ausgebildet ist.

7. Dispenser nach einem der voranstehenden Ansprüche, **dadurch gekennzeichnet, dass** der Dispenser (1) ein Erfassungselement (14) aufweist, das zumindest teilweise in eine axial ausgerichtete Aussparung (15) in einer Stirnseite des Kolbens (5) in Axialrichtung (A) einführbar ist,
dass das zweite Positionselement (11) bzw. der Positionsmarker (11) bewegungsmäßig mit dem Erfassungselement (14) gekoppelt ist und
dass mittels der Einrichtung zur Positionsbestimmung (9) die Tiefe der Aussparung (15) bestimmbar ist.

8. System zum Aufnehmen und Abgeben von Fluidvolumina mit einer als Austauschteil ausgebildeten Kolben-Zylinder-Einheit (3) und einem Dispenser (1) gemäß einem der Ansprüche 1 bis 7, wobei
- die Kolben-Zylinder-Einheit (3) am Dispenser (1) lösbar angebracht ist,
- die Kolben-Zylinder-Einheit (3) einen Kolben (5) und einen am Dispenser (1) fixierten Zylinder (6) aufweist und
- der Dispenser (1) ein Kolbenstellglied (8) aufweist, das mit dem Kolben (5) derart lösbar verbunden ist, dass der Kolben (5) zum Aufnehmen und Abgeben von Fluidvolumina mittels des Kolbenstellglieds (8) linear verschiebbar ist.

9. Verfahren zum Aufnehmen und Abgeben von einstellbaren Fluidvolumina mittels eines Dispensers (1) gemäß einem der Ansprüche 1 bis 7, an dem eine als Austauschteil ausgebildete Kolben-Zylinder-Einheit (3) lösbar anbringbar ist, wobei - die Kolben-Zylinder-Einheit (3) einen Kolben (5) und einen Zylinder (6) aufweist und
wobei das Verfahren die folgenden Verfahrensschritte umfasst:
a) Lösbares Anbringen der Kolben-Zylinder-Einheit (3) am Dispenser (1), vorzugsweise mittels einer zumindest im Wesentlichen in Axialrichtung (A) des Dispensers (1) verlaufenden Bewegung;
b) Lösbares Verbinden des Kolbens (5) mit dem Kolbenstellglied (8), so dass der Kolben (5) zum Aufnehmen und Abgeben von Fluidvolumina mittels des Kolbenstellglieds (8) linear verschiebbar ist;
c) Erzeugen einer Relativbewegung zwischen dem Kolben (5) und dem Zylinder (6) mittels des Kolbenstellglieds (8), indem das Kolbenstellglied (8) den Kolben (5) linear verschiebt;
d) Inkrementelles Bestimmen des zurückgelegten Wegs des Kolbens (5) relativ zum Gehäuse (2) des Dispensers (1), wobei mittels einer Drehmesseinrichtung des Dispensers (1) die Umdrehungen eines das Kolbenstellglied (8) antreibenden Motors (12) des Dispensers (1) bestimmt werden und somit die Länge des linearen Verschiebeweges des Kolbens (5) im Zylinder (6) bei Verstellung des Kolbenstellglieds (8) inkrementell bestimmt wird, während das Kolbenstellglied (8) durch den Motor (12) bewegt wird, und
e) Kontinuierliches Erfassen der absoluten Wegposition des zweiten Positionselements (11) des Dispensers (1) und somit des Kolbenstellglieds (8).

10. Verfahren nach Anspruch 9, wobei nach Schritt a) die Fertigstellung eines erfolgreichen Anbringens der Kolben-Zylinder-Einheit (3) am Dispenser (1) erfasst wird, vorzugsweise mittels einer Lichtschranke (22).

11. Verfahren nach einem der Ansprüche 9 bis 10, wobei anhand erfasster Positionen des zweiten Positionselements (11) eine Richtungsumkehr der Relativbewegung zwischen dem Kolben (5) und dem Zylinder (6) erfasst wird.

12. Verfahren nach einem der Ansprüche 9 bis 11, wobei mindestens ein inkrementell bestimmter zurückgelegter Weg des Kolbens (5) in Bezug gesetzt wird zu mindestens einer erfassten Position des zweiten Positionselements (11) und auf dieser Basis eine Justierung der Wegmessung und/oder der Positionserfassung vorgenommen wird.

13. Verfahren nach einem der Ansprüche 10 bis 12, wobei ein kolbenseitiger Referenzpunkt erfasst wird, indem das Kolbenstellglied (8) des Dispensers (1) auf den Kolben (5) zu bewegt wird, bis ein Anschlag (13) des Kolbenstellglieds (8) an einer Stirnseite des Kolbens (5) anliegt, vorzugsweise wobei der dabei vom Kolbenstellglied (8) zurückgelegte Weg bestimmt wird.

14. Verfahren nach einem der Ansprüche 10 bis 13, wobei das zweite Positionselement (11) bewegungsmäßig mit einem Erfassungselement (14) des Dispensers (1) gekoppelt ist und anhand erfasster Positionen des zweiten Positionselements (11) die Position des Erfassungselements (14) relativ zum Gehäuse (2) des Dispensers (1) bestimmt wird.

15. Verfahren nach Anspruch 14, wobei das Erfassungselement (14) des Dispensers (1) zumindest teilweise in eine axial ausgerichtete Aussparung (15) des Kolbens (5) in Axialrichtung (A) eingeführt und die Tiefe dieser Aussparung (15) anhand erfasster Positionen des zweiten Positionselements (11) bestimmt und der bestimmte Tiefenwert zur Identifizierung des Typs der Kolben-Zylinder-Einheit (3) verwendet wird.

## Claims

1. Dispenser for taking up and dispensing fluid volumes, to which dispenser a piston-cylinder unit (3), which is realized as an exchangeable part and has a piston (5) and a cylinder (6), is releasably attachable,
wherein the dispenser (1) comprises a housing (2) and an incremental distance measuring device (7) which is constructed such that when there is a relative movement between the piston (5) and the cylinder (6), the distance traveled by the piston (5) relative to the housing (2) of the dispenser (1) is determinable in an incremental manner,
wherein the dispenser (1) comprises a piston actuator (8) which is releasably connectable to the piston (5) in such a manner that the piston (5) is linear movable by means of the piston actuator (8) for taking up and/or dispensing fluid volumes, wherein the incremental distance measuring device (7) has a rotary measuring device by means of which the revolutions of a motor (12) of the dispenser (1) operating the piston actuator (8) can be determined and thus the length of the linear displacement path of the pistons (5) in the cylinder (6) can be determined incrementally when the piston actuator (8) is adjusted,
**characterized in that**
the dispenser (1) comprises a position determining device (9) which has a first position element (10) and a second position element (11),
the first position element (10) is arranged rigidly in and/or on the housing (2) of the dispenser (1) and the second position element (11) is motionally coupled with the piston actuator (8) and
the position determining device (9) is constructed for continuous detection of the absolute displacement position of the second position element (11).

2. Dispenser as claimed in claim 1, **characterized in that** the first position element (10) is a position sensor and the second position element (11) is a position marker and the position sensor (10) is constructed to continuously determine the absolute displacement position of the position marker (11), preferably wherein the position sensor (10) comprises a printed circuit board.

3. Dispenser as claimed in claim 2, **characterized in that** the position marker (11) comprises an electrical resonator, preferably with a coil and/or a capacitor.

4. Dispenser as claimed in claim 1, **characterized in that** the first position element (10) is a position indicator with at least one measuring body and the second position element (11) is a position sensor with at least one sensor and the position sensor (11) is constructed to continuously determine its absolute displacement position, namely to read its position from the position indicator (10).

5. Dispenser as claimed in one of the preceding claims, **characterized in that** the position determining device (9) is constructed for the optical, inductive and/or capacitive, preferably resonantly inductive, determination of the position of the second position element (11).

6. Dispenser as claimed in one of the preceding claims, **characterized in that** the position determining device (9) is constructed to determine the position of the second position element (11) without mechanical and without electrical contact between the first position element (10) and the second position element (11).

7. Dispenser as claimed in one of the preceding claims, **characterized in that** the dispenser (1) comprises an acquisition element (14) which is introducible in the axial direction (A) at least in part into an axially aligned recess (15) in an end face of the piston (5),
**in that** the second position element (11) and/or the position marker (11) is motionally coupled with the acquisition element (14) and
**in that** the depth of the recess (15) is determinable by means of the position determining device (9).

8. System for taking up and dispensing fluid volumes having a piston-cylinder unit (3) which is realized as an exchangeable part and a dispenser (1) as claimed in one of claims 1 to 7, wherein
- the piston-cylinder unit (3) is releasably attached to the dispenser (1),
- the piston-cylinder unit (3) comprises a piston (5) and a cylinder (6) which is fixed on the dispenser (1) and
- the dispenser (1) comprises a piston actuator (8) which is releasably connected to the piston (5) in such a manner that the piston (5) is linear movable by means of the piston actuator (8) for taking up and dispensing fluid volumes.

9. Method for taking up and dispensing adjustable fluid volumes by means of a dispenser (1) as claimed in one of the claims 1 to 7, to which a piston-cylinder unit (3), which is realized as an exchangeable part, is releasably attachable, wherein the piston-cylinder unit (3) comprises a piston (5) and a cylinder (6) and
wherein the method includes the following method steps:
a) releasably attaching the piston-cylinder unit (3) to the dispenser (1), preferably by means of a movement which extends at least substantially in the axial direction (A) of the dispenser (1);
b) releasably connecting the piston (5) to the piston actuator (8) so that the piston (5) is linearly displaceable by means of the piston actuator (8) for receiving and dispensing fluid volumes;
c) generating a relative movement between the piston (5) and the cylinder (6) by means of the piston actuator (8) by the piston actuator (8) displacing the piston (5) linearly;
d) incrementally determining the distance traveled by the piston (5) relative to the housing (2) of the dispenser (1), wherein the revolutions of a motor (12) of the dispenser (1) operating the piston actuator (8) are determined by means of the rotary measuring device of the dispenser (1) and thus the length of the linear displacement path of the piston (5) in the cylinder (6) is determined incrementally when the piston actuator (8) is adjusted while the piston actuator (8) is moved by the motor (12), and
e) continuously determining the absolute displacement position of the second position element (11) of the dispenser (1) and consequently of the piston actuator (8).

10. Method as claimed in claim 9, wherein after step a) the completion of a successful attachment of the piston-cylinder unit (3) to the dispenser (1) is detected, preferably by means of a light barrier (22).

11. Method as claimed in one of claims 9 to 10, wherein a reversal in the direction of the relative movement between the piston (5) and the cylinder (6) is determined by way of determined positions of the second position element (11).

12. Method as claimed in one of claims 9 to 11, wherein at least one incrementally determined distance traveled by the piston (5) is related to at least one determined position of the second position element (11) and an adjustment of the displacement measurement and/or position detection is made on said basis.

13. Method as claimed in one of claims 10 to 12, wherein a piston-side reference point is determined by the piston actuator (8) of the dispenser (1) being moved toward the piston (5) until a stop (13) of the piston actuator (8) abuts against an end face of the piston (5).

14. Method as claimed in one of claims 10 to 13, wherein the second position element (11) is motionally coupled with an acquisition element (14) of the dispenser (1) and the position of the acquisition element (14) relative to the housing (2) of the dispenser (1) is determined by way of determined positions of the second position element (11).

15. Method as claimed in claim 14, wherein the acquisition element (14) of the dispenser (1) is introduced in the axial direction (A) at least in part into an axially aligned recess (15) of the piston (5) and the depth of said recess (15) is determined by way of determined positions of the second position element (11) and the determined depth value is used to identify the type of the piston-cylinder unit (3).

## Revendications

1. Distributeur pour la réception et la distribution de volumes de fluides, auquel peut être fixé de manière amovible une unité piston-cylindre (3) conçue comme une pièce d'échange avec un piston (5) et un cylindre (6),
dans lequel le distributeur (1) comporte un boîtier (2) et un dispositif de mesure incrémentielle du déplacement (7) qui est conçu de telle sorte que, en cas de mouvement relatif entre le piston (5) et le cylindre (6), la distance parcourue par le piston (5) par rapport au boîtier (2) du distributeur (1) peut être déterminée de manière incrémentielle,
dans lequel le distributeur (1) comporte un actionneur de piston (8) qui peut être relié de manière amovible au piston (5) de telle sorte que le piston (5) peut être déplacé linéairement au moyen de l'actionneur de piston (8) pour recevoir et distribuer des volumes de fluide,
dans lequel le dispositif de mesure incrémentielle du déplacement (7) comporte un dispositif de mesure de la rotation au moyen duquel on peut déterminer les tours d'un moteur (12) du distributeur (1) entraînant l'actionneur de piston (8) et donc la longueur de la trajectoire de déplacement linéaire du piston (5) dans le cylindre (6) peut être déterminée de manière incrémentielle lors du déplacement de l'actionneur de piston (8),
**caractérisé en ce que**
le distributeur (1) comprend un dispositif de détermination de la position (9) ayant un premier élément de position (10) et un second élément de position (11),
le premier élément de position (10) est disposé de manière rigide dans et/ou sur le boîtier (2) du distributeur (1) et le second élément de position (11) est couplé en termes de mouvement à l'actionneur de piston (8),
le dispositif de détermination de la position (9) est conçu pour détecter en continu la position absolue de la trajectoire du deuxième élément de position (11).

2. Distributeur selon la revendication 1, **caractérisé en ce que** le premier élément de position (10) est un capteur de position et le second élément de position (11) est un marqueur de position et le capteur de position (10) est conçu pour détecter en continu la position de la trajectoire absolue du marqueur de position (11), de préférence dans lequel le capteur de position (10) comprend une carte de circuit imprimé.

3. Distributeur selon la revendication 2, **caractérisé en ce que** le marqueur de position (11) comprend un résonateur électrique, de préférence avec une bobine et/ou un condensateur.

4. Distributeur selon la revendication 1, **caractérisé en ce que** le premier élément de position (10) est un indicateur de position avec au moins un corps de mesure, et le deuxième élément de position (11) est un capteur de position avec au moins un capteur, et le capteur de position (11) est conçu pour la détection continue de la position absolue de sa trajectoire, notamment pour la lecture de sa position sur l'indicateur de position (10).

5. Distributeur selon l'une des revendications précédentes, **caractérisé en ce que** le dispositif de détermination de la position (9) est conçu pour la détection optique, inductive et/ou capacitive, de préférence inductive résonante, de la position du deuxième élément de position (11).

6. Distributeur selon l'une des revendications précédentes, **caractérisé en ce que** le dispositif de détermination de la position (9) est conçu pour détecter la position du deuxième élément de position (11) sans contact mécanique et sans contact électrique entre le premier élément de position (10) et le deuxième élément de position (11).

7. Distributeur selon l'une des revendications précédentes, **caractérisé en ce que** le distributeur (1) comporte un élément de détection (14) qui peut être inséré au moins partiellement dans un évidement (15) aligné axialement dans une face frontale du piston (5) dans la direction axiale (A),
**en ce que** le deuxième élément de position (11) et/ou le marqueur de position (11) est couplé en termes de mouvement à l'élément de détection (14), et
en ce sens que la profondeur de l'évidement (15) peut être déterminée au moyen du dispositif de détermination de la position (9).

8. Système de réception et de distribution de volumes de fluide comprenant une unité piston-cylindre (3) conçue comme une pièce d'échange et un distributeur (1) selon l'une quelconque des revendications 1 à 7, dans lequel
- l'unité piston-cylindre (3) est fixée de manière amovible au distributeur (1),
- l'ensemble piston-cylindre (3) comprend un piston (5) et un cylindre (6) fixé au distributeur (1), et
- le distributeur (1) comporte un actionneur de piston (8) qui est relié de manière amovible au piston (5) de telle sorte que le piston (5) peut être déplacé linéairement au moyen de l'actionneur de piston (8) pour recevoir et distribuer des volumes de fluide.

9. Procédé de réception et de distribution de volumes de fluide réglables au moyen d'un distributeur (1) selon l'une quelconque des revendications 1 à 7, sur lequel une unité piston-cylindre (3) conçue comme une pièce d'échange peut être fixée de manière amovible, l'unité piston-cylindre (3) comportant un piston (5) et un cylindre (6), et
dans lequel le procédé comprend les étapes suivantes :
a) Fixation amovible de l'unité piston-cylindre (3) sur le distributeur (1), de préférence par un mouvement s'étendant au moins essentiellement dans la direction axiale (A) du distributeur (1);
b) Connexion amovible du piston (5) avec l'actionneur de piston (8), de sorte que le piston (5) est déplaçable linéairement au moyen de l'actionneur de piston (8) pour recevoir et distribuer des volumes de fluide;
c) Génération d'un mouvement relatif entre le piston (5) et le cylindre (6) au moyen de l'actionneur de piston (8), en ce sens que l'actionneur de piston (8) déplace le piston (5) de manière linéaire;
d) Détermination incrémentale de la distance parcourue par le piston (5) par rapport au boîtier (2) du distributeur (1), dans lequel les tours d'un moteur (12) du distributeur (1) entraînant l'actionneur à piston (8) sont déterminées au moyen d'un dispositif de mesure de la rotation du distributeur (1) et ainsi la longueur de la trajectoire de déplacement linéaire du piston (5) dans le cylindre (6) est déterminée de manière incrémentielle lors du déplacement de l'actionneur de piston (8) tandis que l'actionneur de piston (8) est déplacé par le moteur (12), et
e) Détection continue de la position de la position absolue de la trajectoire du deuxième élément de position (11) du distributeur (1) et donc de l'actionneur de piston (8).

10. Procédé selon la revendication 9, dans lequel, après l'étape a), l'achèvement d'une fixation réussie de l'unité piston-cylindre (3) sur le distributeur (1) est détecté, de préférence au moyen d'un capteur photoélectrique (22).

11. Procédé selon l'une quelconque des revendications 9 à 10, dans lequel une inversion du sens du mouvement relatif entre le piston (5) et le cylindre (6) est détectée sur la base des positions détectées du deuxième élément de position (11).

12. Procédé selon l'une quelconque des revendications 9 à 11, dans lequel au moins une distance incrémentale déterminée parcourue par le piston (5) est liée à au moins une position détectée du deuxième élément de position (11) et, sur cette base, un ajustement de la mesure de distance et/ou de la détection de position est effectué.

13. Procédé selon l'une des revendications 10 à 12, dans lequel un point de référence du côté du piston est détecté en déplaçant l'actionneur de piston (8) du distributeur (1) vers le piston (5) jusqu'à ce qu'une butée (13) de l'actionneur de piston (8) s'appuie contre une face frontale du piston (5), de préférence en déterminant la distance parcourue par l'actionneur de piston (8) dans le processus.

14. Procédé selon l'une quelconque des revendications 10 à 13, dans lequel le deuxième élément de position (11) est couplé en termes de mouvement à un élément de détection (14) du distributeur (1) et la position de l'élément de détection (14) par rapport au boîtier (2) du distributeur (1) est déterminée sur la base des positions détectées du deuxième élément de position (11).

15. Procédé selon la revendication 14, dans lequel l'élément de détection (14) du distributeur (1) est inséré au moins partiellement dans un évidement (15) du piston (5) orienté axialement dans la direction axiale (A) et la profondeur de cet évidement (15) est déterminée sur la base des positions détectées du deuxième élément de position (11) et la valeur de profondeur déterminée est utilisée pour identifier le type de l'unité piston-cylindre (3).
